# EUROPÄISCHE PATENTSCHRIFT

(11) **EP 2 138 197 B1**
(45) Veröffentlichungstag und Bekanntmachung des Hinweises auf die Patenterteilung: **22.12.2010**
(21) Anmeldenummer: 08104546.0
(22) Anmeldetag: 26.06.2008
(51) Int. Cl.: A61M 1/06, A61M 1/00

(54) **Milchpumpe**
Milk pump
Pompe à lait

(43) Veröffentlichungstag der Anmeldung: 30.12.2009
(73) Patentinhaber: Trimed AG, 9495 Triesen (LI)
(72) Erfinder: Nüesch, Hansueli, 5453 Remetschwil (CH)
(74) Vertreter: Stocker, Kurt

(56) Entgegenhaltungen:
- EP-A- 1 818 067
- WO-A-2004/000390
- US-A- 5 358 476

## Beschreibung

Die Erfindung bezieht sich auf eine Milchpumpe nach dem Oberbegriff des Anspruches 1.

Eine derartige Milchpumpe ist beispielsweise aus der EP-A-1 818 067 bekannt geworden. Dabei wird ein Saugorgan in Form einer etwa topf- oder schalenförmigen Membrane innerhalb des Hubraumes von einem Betätigungshebel bewegt. Besonders berufstätige Mütter pumpen so Muttermilch ab, um sie dem Säugling während des Tages, während welcher Zeit das Kind von einer Amme oder einer Verwandten betreut wird, zur Verfügung zu stellen.

Es ist bekannt, dass berufstätige Mütter einer grossen Stressbelastung ausgesetzt sind. Das Abpumpen der Muttermilch nimmt eine gewisse Zeit in Anspruch, was die morgendliche Stress-Situation noch verschärft. Obgleich nun aber Handpumpen der oben genannten Art preislich relativ günstig sind, verzichten manche Frauen darauf und wählen lieber die teureren Motorpumpen. Als Grund wird vielfach die rasche Ermüdung der Hand angegeben.

Eine solche Ermüdung stellt sich besonders dann ein, wenn die Pumpe kurzhubig ist und daher viele Pumpbewegungen nötig sind, um einen angemessenen Saugeffekt zu erzielen. Dies gilt besonders etwa für Konstruktionen mit relativ kurzen Membranen, beispielsweise der einen Kolben abdichtenden Rollmembrane nach der WO 2004/058330 oder der EP-0 733 376, deren Hub eben ziemlich begrenzt ist, so dass sie öfter betätigt werden müssen, um dasselbe Ergebnis wie etwa eine Kolbenpumpe zu erreichen. Zudem sind solche Membrane auf Zug relativ empfindlich und können leicht reissen, weil sie im allgemeinen entgegen ihrer Eigenelastizität bewegt werden.

Da das Überlaufen der Milch in den Pumpraum verhindert werden muss, ist es auch bekannt geworden (vgl. WO 2006/000292), eine Membrane über den gesamten Saugquerschnitt als Dichtung einzusetzen und diese Membrane indirekt durch einen Kolben und dessen Saugwirkung zu bewegen. Allerdings ist diese Konstruktion ziemlich platzaufwendig und hat auch den Nachteil, dass die Kolbenbewegung mit der Membranbewegung synchron ablaufen muss; wird etwa die Membrane beim Einsetzen in die Pumpe (was zum Waschen nach Gebrauch erforderlich ist) etwas gequetscht eingesetzt, wogegen der Kolben den ersten Hub in voller Länge ausführt, dann kann eine effiziente und leichte Pumparbeit nicht mehr geleistet werden.

All diese Umstände werden auch in der schon genannten EP-A-1 818 067 geschildert, und es wird vorgeschlagen die Membrane so zu führen, dass sie praktisch nach allen Seiten leicht beweglich ist, um Reibung und Widerstand durch etwa an der Membrane oder der Innenseite des Hubraumes anklebende Milch zu vermeiden. Dies bedeutet aber dass nicht der ganze Hubraum für das Ansaugen durch das Saugorgan genutzt werden kann. Zwar ist es etwa aus der WO 03/013628 oder (für Motorpumpen, wo der Antrieb über einen Exzenter erfolgt) durch die US-A-2001/0038799 bekannt, eine etwa kalottenförmige Membrane dicht in einen etwa kalottenförmigen Hubraum einzusetzen, um so das gesamte durch den Hubraum gegebene Volumen zum Pumpen auszunützen. Auch wurde schon vorgeschlagen, ringförmig um die Bewegungslängsachse der Membrane verlaufende Verdickungen bzw. Rippen vorzusehen, um eine Vergleichmässigung der Membranverformung zu erzielen.

Der vorliegenden Erfindung liegt die Aufgabe zugrunde, eine Handpumpe der eingangs genannten Art weiter zu verbessern und ihren konstruktiven Aufbau zu vereinfachen, ohne dass es dabei zu einer Erschwernis beim Abpumpen der Muttermilch käme. Erfindungsgemäss gelingt dies durch die kennzeichnenden Merkmale des Anspruches 1.

Durch die Erfindung ergibt sich eine ganze Reihe von Vorteilen:
- durch die schraubenlinienförmige Ausbildung ergibt sich (je nach Wahl) eine mehr oder minder ausgeprägte Federwirkung, welche mindestens Teil der Rückholanordnung, wenn sie sie nicht sogar ganz ersetzt. Dabei kann die Federwirkung durch Wahl des Materiales und der Dicke bzw. Dimension der schraubenlinienartigen Verdickung beeinflusst werden. So kann gewünschtenfalls auf eine zusätzliche Rückholeinrichtung verzichtet werden;
- gleichzeitig ergibt sich eine gewisse Abstandhalterwirkung gegenüber der Innenwand des Hubraumes, denn ist die Schraubenlinie im Inneren der als Hohlkörper ausgebildeten Membrane angeordnet, dann wird sie die Membrane an der Verbindungsstelle mit der Membranwand nach aussen durchdrücken und so eine äussere , schraubenlinienförmige Rippe bilden; bevorzugt ist sie jedoch im Sinne des Anspruches 3 an der Aussenseite angebracht;
- ausserdem erfüllt auch die schraubenlinienförmige Ausbildung die Funktion einer Vergleichmässigung des Zusammenfaltens der Membrane, so dass Verklemmungen und Störungen praktisch ausgeschlossen sind;
- da die Schraubenfeder so zusammen mit der Membrane hergestellt wird, ist die Herstellung der Rückholanordnung vereinfacht und kostengünstiger;
- ferner sind zur Herstellung einer solchen Membrane in der Spritzgiessmaschine im allgemeinen keine Kernzüge erforderlich, und die Entformung ist erleichtert;
- schliesslich wird durch die Abstandhalterfunktion der Schraubenlinie das Ankleben der Membrane durch Saugwirkung und/oder durch Anhaften an den einander gegenüberliegenden Wänden von Hubraum und Membrane etwa infolge dort klebender Milch (falls diese unbeabsichtigt in den Hubraum gelangt ist) verhindert.

Weitere Einzelheiten der Erfindung ergeben sich an Hand der nachfolgenden Beschreibung von in der Zeichnung schematisch dargestellten Ausführungsbeispielen. Es zeigen:
- Fig. 1: eine Perspektivansicht einer erfindungsgemässen Milchpumpe;
- Fig. 2: eine explodierte Darstellung des Hubraumes und der darin untergebrachten Teile der Milchpumpe gemäss Fig. 1;
- Fig. 3: einen Längsschnitt durch eine zusammengebaute Milchpumpe nach einem weite- ren Ausführungsbeispiel;
- Fig. 4: eine erfindungsgemässe Membrane, wie sie bei einer Ausführungsform gemäss Fig. 2 bevorzugt zur Anwendung kommen kann, und die
- Fig. 5 und 6: zwei Ausführungen einer Membrane, wie sie bei einer Ausführungsform gemäss Fig. 3 zur Anwendung kommen kann.

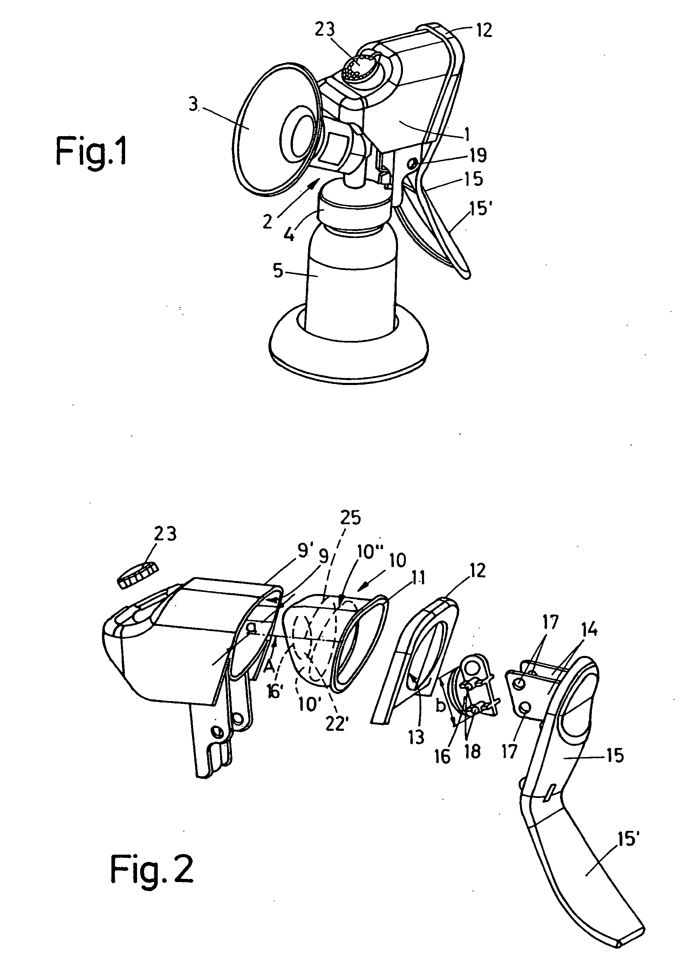

Die in den Fig. 1 und 2 dargestellte Milchpumpe weist einen Oberteil 1 (Handpumpeneinheit) auf, der auf einen Verbindungsteil 2 aufgesteckt ist. Der Verbindungsteil 2 umfasst einerseits ein Brustansatzstück 3 oder Horn, anderseits einen Schraubteil 4 mit dem die Pumpe auf einen Milchsammelbehälter 5 aufschraubbar ist. Zwischen Milchsammelbehälter 5 und dem Verbindungsteil 2 liegt zweckmässig in bekannter Weise ein Rückschlagventil. An der Oberseite mag ein bekannter Reglerknopf 23 angebracht sein, der die Zufuhr von Falschluft regelt und damit auch die Mühe bei der Pumpbetätigung. Eine besonders bevorzugte Ausführung ist beispielsweise in den US-A-6,042,560 und 6,290,671 beschrieben.

Innerhalb des Oberteils 1 ist ein, am besten aus Fig. 2 ersichtlicher Hubraum 9 vorgesehen, der von einer Wandung 9' umgeben ist. Die Wandung 9' hat, wie besonders Fig. 2 zeigt, einen im wesentlichen viereckigen Querschnitt mit abgerundeten Ecken, könnte aber auch einen zylindrischen Querschnitt aufweisen. In diesen Hubraum 9 ist eine etwa topfförmige Membrane 10 eingesetzt und mit einem ihren Rand 11 festhaltenden Rahmen 12 an einem Ende der den Hubraum 9 begrenzenden Wandung 9' befestigt. Diese Membrane 10 besteht vorteilhaft aus einem Silikon-Kunststoff, der einerseits lebensmitteltauglich ist, anderseits auch die hier besonders geforderte Elastizität gewährleistet.

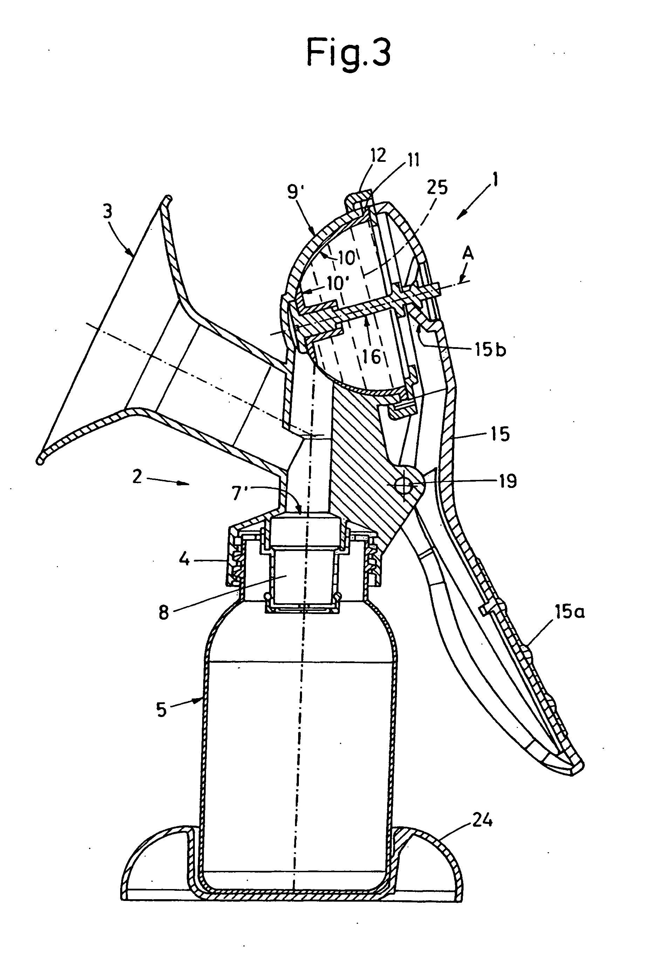

Der Rahmen 12 besitzt eine Durchlassöffnung 13, durch welche ein an zwei Vorsprüngen 14 eines zweiarmigen Betätigungshebels 15 befestigtes, etwa scheibenförmiges, Führungsschild 16 angebracht ist. Hierzu weisen die Vorsprünge 14 gemäss Fig. 3 Löcher 17 auf, in welche Stifte oder Vorsprünge 18 am Führungsschild 16 einsetzbar und/oder einschnappbar sind.

Fig. 2 veranschaulicht das Führungsschild 16, an dem die etwa topfförmige Membrane 10 befestigt ist. Zu diesem Zweck weist die Membrane 10 einen sich quer zum Hubraum 9 erstreckenden Abschnitt 10' (strichliert in Fig. 2) mit einer das Führungsschild 16 aufnehmenden Öffnung 16' (vgl. Fig. 2) und einen - im durch den Hubraum 9 gestreckten Zustand (vgl. Fig. 2) - sich etwa in Umfangsrichtung der Wandung 9' erstreckenden Abschnitt 10" auf. Der Abschnitt 10' bildet also sozusagen die Bodenfläche der topfförmigen Membrane 10, wogegen der Abschnitt 10" die (mindestens eine) Umfangsfläche bildet. Im Inneren der hohlen, etwa topfförmigen Membrane 10 sind schraubenlinienförmig verlaufende Verdickungen 25, welche einerseits - infolge der Elastizität des Materials - eine Federwirkung ausüben, anderseits aber die Membrane 10 in radialer Richtung versteifen, so dass ihre Aussenseite an der Innenwand des Hubraumes 9 anliegt und somit gegen Eindringen von Milch abdichtet, es aber auch gleichzeitig erlaubt, dass die dazwischen liegende Membranhaut sich von der Innenwand des Hubraumes 9 abhebt und so ein Anhaften oder Ankleben (welches die Pumparbeit erschweren würde) der Membrane 10 an der Innenwand des Hubraumes vermieden wird.

Da der Hubraum 9 eine geradlinige Bewegungslängsachse A besitzt, das Führungsschild 16 dagegen mit dem Hebel 15 verbunden ist und mit diesem um eine Schwenkachse 19 (Fig. 1, 2) schwenkt, ergibt sich - wie aus Fig. 2 strich-punktiert ersichtlich - bei Druck auf das untere Ende des Hebels 15 eine etwa kreisförmige Bewegung.

Die in Fig. 3 dargestellte Milchpumpe steht mit ihrem Milchbehälter 5 zweckmässig in einem die Standfläche vergrössernden Fussteil 24. Innerhalb des Oberteils 1 ist wiederum ein Hubraum 9 vorgesehen, der von einer Wandung 9' umgeben ist. Die Wandung 9' hat hier vorzugsweise eine im wesentlichen kugelkalottenartige Form. In diesen Hubraum 9 ist eine vorteilhaft komplementär geformte kalottenförmige Membrane 10 aus verformbarem bzw. elastischem Material eingesetzt und mit einem ihren Rand 11 festhaltenden Rahmen 12 an einem Ende der den Hubraum 9 begrenzenden Wandung 9' befestigt. Die Kalottenkrümmung von Hubraum 9 und Membrane 10 sind also im wesentlichen hinsichtlich Umfang, Krümmung und axialer Länge in Richtung der Achse A gleich dimensioniert.

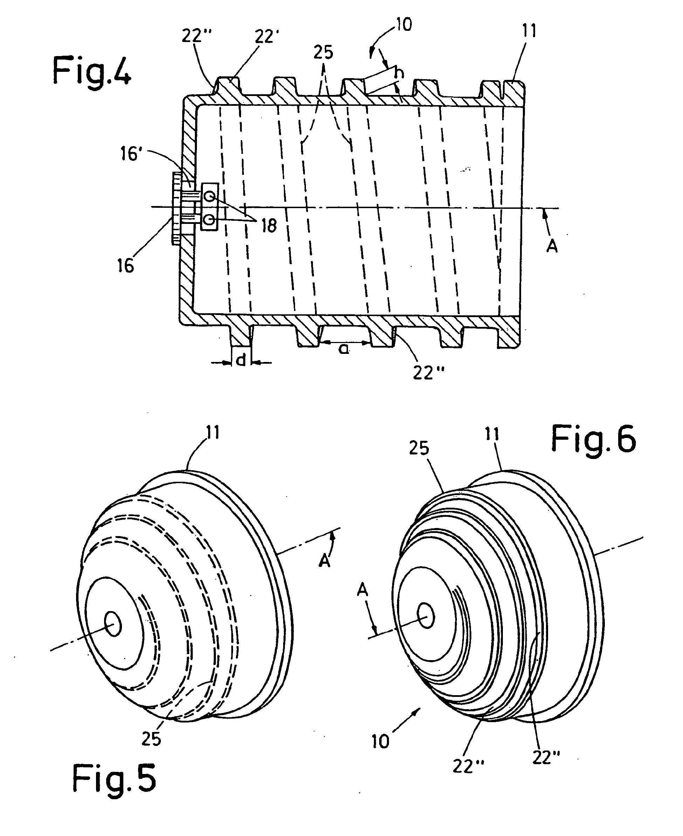

Anderseits ist aus Fig. 3 ersichtlich, dass die Dicke der Membrane 10 vorzugsweise in axialer Richtung (Achse A) variiert. Damit lässt sich zwar einerseits die Federcharakteristik beeinflussen, anderseits wird dadurch ein gleichmässiges Falten der Membrane bei Betätigung des Hebelarmes 15a begünstigt. Um die Federcharakteristik auch im Sinne einer Verstärkung beeinflussen zu können, sind, beispielsweise im Inneren der hohlen Membrane 10, schraubenlinienförmige Rippen bzw. Verdickungen 25 Fig. 2, 3) vorgesehen, welche so eine Art Schraubendruckfeder bilden, und die später an Hand der Fig. 4 bis 6 noch im einzelnen besprochen werden. Dies ermöglicht es, mindestens zum Teil auf eine gesonderte Rückholanordnung für die Betätigungseinrichtung bzw. den Hebel 15 bzw. das Saugorgan 10 zum Rückholen in eine vorbestimmte Ausgangslage zu verzichten und so die Gesamtkonstruktion der Milchpumpe zu vereinfachen.

Auch im Falle der Fig. 3 besitzt der Rahmen 12 wiederum eine Durchlassöffnung 13, durch welche ein an einem zweiarmigen, um eine Achse 19 schwenkbaren Betätigungshebel 15 mit Grifffläche 15a befestigter, etwa stiftförmiger Führungsteil 16 angebracht ist, dessen freies Ende mit dem sich quer zum Hubraum 9 und seiner Achse A erstreckenden, mittleren Abschnitt 10' verbunden ist (vgl. Fig. 2). Der Führungsteil 16 ist an einer Haltefläche 15b des Hebels 15 eingehängt, so dass er gegebenenfalls auch leicht demontierbar ist.

Fig. 4 zeigt ein gegenüber der Ausführung nach Fig. 2 bevorzugtes Ausführungsbeispiel für eine etwa zylindrische bzw. topfförmige Membrane 10. Anders als bei der Ausführung nach Fig. 2 sind eine Schraubenlinie 25 bildende Verdickungen 22' an der Aussenseite der Membrane 10 angebracht, was besonders deswegen bevorzugt ist, weil damit noch sicherer ein Anhaften der Membrane an der Innenwand des Hubraumes vermieden wird.

Es ist ersichtlich, dass zwischen den einzelnen Schraubenwindungen ein relativ grosser Abstand a vorhanden ist. Es kann erwünscht sein, diesen Abstand a zur besseren Abdichtung gegen eindringende Milch zu verkleinern. Dazu ist es entweder möglich, die Schraubenlinien eines einzigen Schraubenganges entsprechend enger zu gestalten oder ein mehrgängige Schraubenlinie vorzusehen, in welch letzterem Falle - bei gleicher Materialwahl und Dimensionierung der Schraubengänge - der Federeffekt noch verstärkt würde. Die Höhe h der Verdickungen 22' bzw. der Schraubenlinie 25 bestimmt den Abstand zur Innenwand des Hubraumes 9, der einerseits nicht zu gross gewählt sein soll, um mit der Pumpbewegung ein möglichst grosses Volumen pumpen zu können, anderseits nicht so klein sein sollte, dass etwa eindringende Milch eine Verklebung mit der Membranwand zwischen den Gängen der Schraubenlinie 25 bewirken könnte.

Dies zeigt, dass es die Erfindung dem Fachmanne ermöglicht, je nach Bedarf die unterschiedlichsten Pump- und Federcharakteristiken zu erzielen, wobei die Bemessung der Dicke d der Schraubenlinie für die Stärke der Federwirkung eine wichtige Rolle spielt. Ferner ist in Fig. 4 zwar eine Schraubenlinie 25 mit Verdickungen 22' etwa viereckigen Querschnitts gezeigt, die Flankenflächen 22" besitzen, doch versteht es sich, dass etwa auch runde Querschnitte - ähnlich dem Querschnitt einer Schraubenfeder - denkbar sind.

Analog zu den Ausbildungen einer im wesentlichen zylindrischen, topfförmigen Membrane 10 gemäss den Fig. 2 und 4 veranschaulichen die Fig. 5 und 6 eine kugelkalottenförmige, becher- oder schalenförmige Membrane 10, wie sie für eine Milchpumpe nach Fig. 3 zum Einsatz kommen kann. Während dabei die Fig. 5 die Ausbildung mit einer im Inneren der hohlen Membrane 10 liegenden (und daher strichliert gezeigten) Schraubenlinie 25 zeigt, welche einen ähnlichen Effekt haben wird, wie dies oben mit Bezug auf die Membrane der Fig. 2 geschildert wurde, die ebenfalls eine im Inneren liegende Schraubenlinie besitzt, stellt die Fig. 6 die bevorzugte Ausführungsform mit einer an der Aussenseite der Membrane 10 angebrachten Schraubenlinie 25 dar, welche dementsprechend auch die oben bereits besprochenen Flankenflächen 22' besitzt. Die beiden Figuren 5 und 6 veranschaulichen auch, dass die jeweilige Schraubenlinie 25 nicht über die ganze axiale Länge (entlang der Achse A) verlaufen muss, so dass damit für den Fachmann eine weitere Möglichkeit besteht, die Pump- und Federcharakteristik zu beeinflussen.

Im Rahmen der Erfindung sind zahlreiche Modifikationen möglich; so wurde oben auf die verschiedenen Beeinflussungsmöglichkeiten der Federcharakteristik hingewiesen, aber es gibt deren weitere. Beispielsweise können die Schraubenlinien innen und aussen angebracht sein, also etwa eine Kombination der Fig. 5 und 6 darstellen, wobei dikese Kombination entweder so gestaltet ist, dass die innere Schraubenlinie gleichsam eine Verstärkung der äusseren ist, d.h. parallel verläuft, oder sie bildet innen einen zweiten, gesonderten Schraubengang. Auch kann eine Beeinflussung der Charakteristik über die Länge des Federweges dadurch erfolgen, dass die Verdickungen 22 oder 22' über ihre Längserstreckung unterschiedlich stark (Breite d) oder ihre Beabstandung a über die Länge (entlang der Achse A) variiert.

Auch ist als Vorteil noch zu erwähnen, dass die Federwirkung selbst dann die Rückstellung der Membrane 10 aus der zusammengepressten Lage in die entspannte Lage unterstützt, wenn die Schraubenlinie lokal an der Innenwand des Hubraumes 9 anhaften sollte, weil die anhaftende Fläche dann nur klein ist (im Vergleich mit der gesamten Aussenfläche der Membrane 10), die Federkraft aber - wie oben erläutert - je nach Bedarf beliebig gestaltet werden kann.

Es versteht sich, dass in der obigen Beschreibung Teile identischer Funktion bei allen Ausführungsformen dieselben Bezugszeichen haben, Teile ähnlicher Funktion dieselben Bezugszeichen, aber mit einem Zusatz. Auch ist klar, dass die Merkmale der einzelnen Ausführungsformen untereinander sowie mit Merkmalen des Standes der Technik austauschbar sind.

## Patentansprüche

1. Membrane (10), welche einen Hohlkörper mit über ihren Verlauf unterschiedlicher Wandstärke bildet, für eine Milchpumpe mit einer Handpumpeneinheit (1), welche einen im wesentlichen von wenigstens einer Wandung (9') umschlossenen, an ein Brustansatzstück (3) angeschlossenen oder anschliessbaren, eine Bewegungslängsachse (A) definierenden, die einen Hohlkörper bildende Membrane mit über ihren Verlauf unterschiedlicher Wandstärke als Saugorgan im wesentlichen umschliessenden Hubraum (9) aufweist, wobei die Membrane als Saugorgan darin mittels einer, insbesondere manuellen, Betätigungseinrichtung (15), wie einem Hebel (15), vorzugsweise einen zweiarmigen, in einem Mittelbereich (bei 19) schwenkbar gelagerten Hebel (15), hin- und herbewegbar ist, und eine Rückholanordnung für die Betätigungseinrichtung (15) bzw. das Saugorgan zum Rückholen in eine vorbestimmte Ausgangslage vorgesehen ist, **dadurch gekennzeichnet, dass** der Verlauf unterschiedlicher Wandstärken der Membrane (10) als sich entlang und koaxial zur Bewegungslängsachse (A) erstreckende schraubenlinienförmige, und somit eine Feder bildende Verdickung (22) ausgebildet ist.

2. Membrane nach Anspruch 1, **dadurch gekennzeichnet, dass** die Schraubenlinie (25) an der Aussenseite des Hohlkörpers (10) ausgebildet ist.

3. Membrane nach Anspruch 1 oder 2, **dadurch gekennzeichnet, dass** die Schraubenlinie (25) mindestens zweigängig ist.

4. Membrane nach einem der vorhergehenden Ansprüche, **dadurch gekennzeichnet, dass** sie (10) aus einem Silikon-Kunststoff besteht.

5. Milchpumpe mit einer Handpumpeneinheit (1), welche einen im wesentlichen von wenigstens einer Wandung (9') umschlossenen, an ein Brustansatzstück (3) angeschlossenen oder anschliessbaren, eine Bewegungslängsachse (A) definierenden, eine einen Hohlkörper bildende Membrane nach einem der vorhergehenden Ansprüche aufweist, welche als Saugorgan in einem sie im wesentlichen umschliessenden Hubraum (9) mittels einer, insbesondere manuellen, Betätigungseinrichtung (15), wie einem Hebel (15), vorzugsweise einen zweiarmigen, in einem Mittelbereich (bei 19) schwenkbar gelagerten Hebel (15), hin- und herbewegbar ist, wobei eine Rückholanordnung für die Betätigungseinrichtung (15) bzw. das Saugorgan zum Rückholen in eine vorbestimmte Ausgangslage

6. Milchpumpe nach Anspruch 5, **dadurch gekennzeichnet, dass** die Schraubenlinie (25) im Inneren des Hohlkörpers, als sich durch die Membrane (10) nach aussen durchdrückende Verdickung (22) ausgebildet ist.

7. Milchpumpe nach Anspruch 5 oder 6, **dadurch gekennzeichnet, dass** die Schraubenlinie (25) an der Aussenseite des Hohlkörpers (10), als einen Abstand (h) zur übrigen Membranwand sichernder Abstandhalter ausgebildet ist.

8. Milchpumpe nach einem der Ansprüche 5 bis 7, **dadurch gekennzeichnet, dass** sich die kalottenförmige Membrane (10) an der Innenwand (9') des kalottenförmigen Hubraums mit der Schraubenlinie (25) abstützt.

## Claims

1. A membrane (10) forming a hollow body which, over its course, has a different wall thickness, for a milk pump including a manual pumping unit (1) presenting a swept volume, which is enclosed substantially within at least one wall (9'), is connected or connectable to a breast catching piece (3), defines a longitudinal axis of movement (A) and substantially surrounds said membrane forming a hollow body which, over its course, has a different wall thickness as a sucking member, said membrane, as the sucking member, may be moved therein to and fro by, particularly manual, actuator means (15), such as a lever (15), preferably a two-armed lever (15) pivotally supported in a middle region (at 19), and a restoring arrangement for the actuator means (15) or the sucking member is provided for restoring it into a predetermined initial position, **characterised in that** the course of different wall thicknesses of the membrane (19) is formed as a helical thickening (22) which extends along and coaxially with the longitudinal axis of movement (A), thus forming a spring.

2. Membrane according to claim 1, **characterised in that** the helical line (25) is formed at the outside of said hollow body (10).

3. Membrane according to claim 1 or 2, **characterised in that** the helical line (25) is at least double-threaded.

4. Membrane according to any of the preceding claims, **characterised in that** it (10) consists of a silicone synthetic material.

5. A milk pump including a manual pumping unit (1) presenting a swept volume, which is enclosed substantially within at least one wall (9'), is connected or connectable to a breast catching piece (3), defines a longitudinal axis of movement (A) and presents a membrane forming a hollow body according to any of the preceding claims, which, as a sucking member, may be moved therein to and fro by, particularly manual, actuator means (15), such as a lever (15), preferably a two-armed lever (15) pivotally supported in a middle region (at 19), wherein a restoring arrangement for the actuator means (15) or the sucking member is provided for restoring it into a predetermined initial position.

6. Milk pump according to claim 5, **characterised in that** the helical line (25) is formed at the inner side of said hollow body as a thickening (22) which squeezes through the membrane (10) to the exterior.

7. Milk pump according to claim 5 or 6, **characterised in that** the helical line (25) is formed at the outside of said hollow body as a spacer which ensures a distance (h) to the rest of the membrane wall.

8. Milk pump according to any of claims 5 to 7, **characterised in that** the dome shaped membrane (10) struts upon the inner wall (9') of the dome shaped swept volume by means of the helical line (25).

## Revendications

1. Membrane (10), qui forme un corps creux ayant une épaisseur de paroi, qui est différente sur son cours, pour une pompe à lait comprenant une unité de pompe à main (1), qui présente une cylindrée (9) sensiblement entourée par au moins une paroi (9'), est attachée ou est attachable à une pièce d'embouchure de buste (3), définit un axe longitudinal de mouvement (A) et entoure sensiblement la membrane formant un corps creux d'une épaisseur de paroi, qui est différente sur son cours, comme un organe d'aspiration, dans laquelle ladite membrane, comme l'organe d'aspiration, est capable de faire un mouvement de va-et-vient au moyen d'un dispositif d'actuation (15), particulirement manuel, comme un levier (15), de préférence un levier (15) à deux bras, qui est logé d'une manière pivotante dans une zone médiane (chez 19), et une disposition de rappel est prévue pour le dispositif d'actuation (15) ou l'organe d'aspiration pour le rappeler dans une position prédéterminée de repos, **caractérisée en ce, que** le cours des épaisseurs différentes de paroi de la membrane (10) est formé comme un épaississement (22) hélicoïdal, qui s'étend le long et coaxialement à l'axe longitudinal de mouvement (A), ainsi formant un ressort.

2. Membrane selon la revendication 1, **caractérisée en ce, que** l'hélice (25) est formée à l'extérieur du corps creux (10).

3. Membrane selon la revendication 1 ou 2, **caractérisée en ce, que** l'hélice (25) est au moins à filet double.

4. Membrane selon une quelconque des revendications précédentes, **caractérisée en ce, qu'**elle (10) consiste d'une matière synthétique à silicone.

5. Pompe à lait comprenant une unité de pompe à main (1), qui présente une cylindrée sensiblement entourée par au moins une paroi (9'), est attachée ou est attachable à une pièce d'embouchure de buste (3), définit un axe longitudinal de mouvement (A) et présente une membrane, qui forme un corps creux, selon une quelconque des revendications précédentes, qui, comme un organe d'aspiration, est capable de faire un mouvement de va-et-vient au moyen d'un dispositif d'actuation (15), particulièrement manuel, comme un levier (15), de préférence un levier (15) à deux bras, qui est logé d'une manière pivotante dans une zone médiane (chez 19), dans une cylindrée (9), qui sensiblement l'entoure, une disposition de rappel étant prévue pour le dispositif d'actuation (15) ou l'organe d'aspiration pour le rappeler dans une position prédéterminée de repos.

6. Pompe à lait selon la revendication 5, **caractérisée en ce, que** l'hélice (25) est formée à l'intérieur du corps creux comme un épaississement (22), qui s'impose à travers la membrane (10) vers l'extérieur.

7. Pompe à lait selon la revendication 5 ou 6, **caractérisée en ce, que** l'hélice (25) est formée à l'extérieur du corps creux (10) comme une cale d'espacement, qui assure une distance (h) au reste de la paroi de membrane.

8. Pompe à lait selon une quelconque des revendications 5 à 7, **caractérisée en ce, que** la membrane (10) en forme de calotte s'appuie par l'hélice (25) à la paroi intérieure (9') de la cylindrée en forme de calotte.
